# EUROPÄISCHE PATENTSCHRIFT

(11) **EP 1 768 580 B1**
(45) Veröffentlichungstag und Bekanntmachung des Hinweises auf die Patenterteilung: **18.09.2013**
(21) Anmeldenummer: 05761607.0
(22) Anmeldetag: 22.06.2005
(51) Int. Cl.: A61B 17/3203, F04B 11/00, F04B 49/22, F04B 53/16

(54) **WASSERSTRAHLCHIRURGIEPUMPE**
WATERJET SURGERY PUMP
POMPE POUR LA CHIRURGIE A JET D'EAU

(30) Priorität: 30.06.2004 DE 102004031673
(43) Veröffentlichungstag der Anmeldung: 04.04.2007
(73) Patentinhaber: ERBE Elektromedizin GmbH, 72072 Tübingen (DE)
(72) Erfinder: MITZLAFF, Lothar, PT-8600-531 Lagos (PT); KÜHNER, Ralf, 70567 Stuttgart (DE); HAGG, Martin, 72827 Wannweil (DE); QUECK, Jochen, 72076 Tübingen (DE)
(74) Vertreter: Bohnenberger, Johannes
(86) Internationale Anmeldenummer: PCT/EP2005/006755
(87) Internationale Veröffentlichungsnummer: WO 2006/002817

(56) Entgegenhaltungen:
- EP-A- 0 551 920
- DE-A1- 4 222 918
- DE-U1- 20 309 616
- US-A- 3 692 052
- US-A- 3 958 898
- US-B1- 6 216 573

## Beschreibung

Die Erfindung betrifft eine Wasserstrahlchirurgiepumpe nach dem Oberbegriff des Anspruches 1.

In der Leberchirurgie wird seit einiger Zeit die Wasserstrahlchirurgie genutzt, da dieses Organ wie kein anderes über Gewebestrukturen unterschiedlicher Festigkeit verfügt (Parenchym, Blutgefäße, Gallengänge) und somit der applizierte Wasserstrahl das zu schneidende Gewebe (Parenchym) zwar durchtrennt, die Blutgefäße und Gallengänge jedoch unbeschädigt lässt. Hierzu ist natürlich eine exakte Steuerung des Schneiddruckes notwendig.

Ein weiteres Problem in der Wasserstrahlchirurgie besteht darin, dass eine absolute Sterilität des Schneidmediums (z.B. Ringer-Lösung) gegeben sein muss, da die Flüssigkeit in die denkbar engste und intensivste Verbindung mit dem Körpergewebe gelangt. Darüber hinaus sind natürlich die üblichen Probleme wie hohe Zuverlässigkeit, Einfachheit und kostengünstige Herstellbarkeit zu beachten.

Aus der US 6,216,573 B1 sowie aus der DE 203 09 616 U1 ist (jeweils) eine medizinische Pumpe für die Wasserstrahlchirurgie bekannt, die auswechselbar, also zur einmaligen Verwendung ausgebildet ist. Die Effizienz der Pumpe sowie ihre Einstellbarkeit genügen jedoch nicht den gestellten Anforderungen.

Aus der EP 0 551 920 A1 ist eine Wasserstrahlchirurgiepumpe mit den Merkmalen des Oberbegriffs von Anspruch 1 bekannt, bei welcher ein Vorratsgefäß vorgesehen ist, das über einen Kolben verschlossen ist. Der Kolben wird über einen Motor derart betätigt, dass die Schneidflüssigkeit unter einen, von der Motor-Kraft vorbestimmten Druck ausgestoßen wird. Die Druckregelung ist aufwändig und ungenau.

Der Erfindung liegt die Aufgabe zu Grunde, eine Wasserstrahlchirurgiepumpe aufzuzeigen, welche trotz einfacher und darum zur einmaligen Verwendung geeigneter Konstruktion verbesserte Schnittleistungen ermöglicht.

Diese Aufgabe wird durch eine Wasserstrahlchirurgiepumpe nach Anspruch 1 gelöst.

Diese Pumpe umfasst ein einstellbares Druckregelventil, um den Druck an einem Fluidauslass auf einen voreinstellbaren Maximalwert zu begrenzen und mindestens zwei Kolben mit Kolbenstangen zum Verschieben der Kolben in Zylindern und zum Ankoppeln an eine Pumpenbetätigungseinrichtung, einen Zylinderkopf zum Abschließen der Zylinder gegenüber der Kolben, Ventileinrichtungen zum Verbinden jeweils eines Druckraumes in den Zylindern mit mindestens einem Fluid-auslass und mindestens einem Fluideinlass, wobei der Fluidauslass mit dem Fluideinlass über ein einstellbares Druckregelventil kommunizierend derart verbunden ist, dass der Druck im Fluidauslass auf einen voreinstellbaren Maximalwert begrenzbar ist, wobei das Druckregelventil den Fluideinlass mit dem Fluidauslass verbindend angeordnet ist.

Dadurch, dass die Pumpe mit zwei Kolben-/Zylindereinrichtungen arbeitet, kann eine verbesserte, insbesondere vergleichmäßigte Förderleistung erzielt werden. Die Konstruktion ist einfach, sodass eine kostengünstige Herstellbarkeit gewährleistet ist. Über das Druckregelventil kann die (ohnehin schon verbesserte) Förderung des Arbeitsfluids nochmals verbessert, insbesondere vergleichmäßigt und gleichzeitig den jeweiligen Anwendungszwecken entsprechend eingestellt werden.

Vorzugsweise umfassen die Ventileinrichtungen und/oder das Druckregelventil eine elastische oder elastisch beaufschlagte Ventil-Membran. Dadurch ist eine sehr kostengünstige Herstellbarkeit bei hoher Betriebssicherheit gewährleistet.

Alternativ können die Ventileinrichtungen zwei federbelastete Kugel-Rückschlagventile umfassen, die ebenfalls in einfacher Weise herstellbar sind.

Das Druckregelventil wird vorzugsweise derart als kraftgesteuertes Ventil ausgebildet, dass der Maximalwert durch eine auf ein Stellglied des Druckregelventils wirkende Stellkraft einstellbar ist. Durch diese besondere Ausbildung ist es in vorteilhafter Weise sehr einfach möglich, die medizinische Pumpe an die Pumpenbetätigungseinrichtung anzukoppeln, wobei eine besonders genaue räumliche Positionierung der Pumpe zur Pumpenbetätigungseinrichtung nicht notwendig ist. Dadurch nämlich, dass die Druckeinstellung nicht wegproportional sondern eben kraftproportional erfolgt, kommt es bei der Ankopplung des Druckregelventils an ein Stellorgan nicht auf dessen Position an (was ein genaues Justieren der Pumpe zur Pumpenbetätigungseinrichtung erfordern würde), vielmehr kommt es lediglich auf die positionsunabhängige Kraft an, mit welcher das Einstellorgan das Druckregelventil betätigt.

Vorzugsweise ist das Druckregelventil zwischen dem Fluideinlass und dem Fluidauslass derart angeordnet, dass beim Übersteigen des Maximalwertes Fluid vom Fluidauslass zum Fluideinlass zurückführbar ist. Auf diese Weise ist ein von der Fördermenge unabhängiges Einstellen des Druckes möglich.

Die Kolben oder die Kolbenstangen sind vorzugsweise über einen Balg, eine Rollmembran oder dergleichen gleitfreie Dichtung mit den Zylindern dicht und keimundurchlässig verbunden. Dadurch kann es nicht geschehen, dass trotz eines sterilen Arbeitsfluids und sterilen Strömungswegen Keime eingeschleppt werden, wie dies bei den bisher bekannten Pumpen durchaus möglich ist. Diese Gefahr ist deshalb besonders hoch, weil durch das Bewegen der Kolben in den Zylindern deren Rückseiten (gegenüber den Druckräumen) beim Pumpen von Umgebungsluft umsttömt und damit die Zylinder in diesem Bereich kontaminiert werden kann.

Vorzugsweise sind die Ventileinrichtungen und/oder das Druckregelventil im Zylinderkopf angebracht. Dadurch ergibt sich ein einfacher, aus wenigen Einzelstücken bestehender Aufbau.

Die Zylinder sind vorzugsweise einzeln mit dem Zylinderkopf verbindbar. Dies erleichtert die Herstellbarkeit.

Vorzugsweise weist der Auslass Verbindungseinrichtungen zum irreversiblen Verbinden mit einem Druckschlauch auf. Dadurch kann gewährleistet werden, dass eine fehlerhafte Installation der Pumpe und auch eine nichtzulässige Wiederverwendung der Pumpe vermieden wird.

Der Zylinderkopf weist vorzugsweise Haltereinrichtungen, insbesondere Haltevorsprünge zum Einrasten von Halteklinken auf, die an der Pumpenbetätigungseinrichtung angebracht sind. Es sind somit keine gesonderten Maßnahmen notwendig, um die Pumpe an der Pumpenbetätigungseintichtung zu montieren.

Bei einer bevorzugten Ausführungsform ist eine Druckspeichereinrichtung vorgesehen und derart ausgebildet und mit dem Fluidauslass verbunden, dass Druckschwankungen des Fluids am Fluidauslass nach Art einer Tiefpassfunktion ausgeglichen werden. Dadurch ergibt sich eine weitere Vergleichmäßigung des Schneidstrahles und somit eine Verbesserung der hier angestrebten Schneidfunktion des Gerätes. Die Druckspeichereinrichtungen sind vorzugsweise im Zylinderkopf angeordnet oder mit diesem verbunden, was den Aufbau der Gesamtanordnung erleichtert.

Bevorzugte Ausführungsformen der Erfindung ergeben sich aus den Unteransprüchen und der nachfolgenden Beschreibung von Ausführungsbeispielen, die anhand von Abbildungen näher erläutert werden. Hierbei zeigen
- Fig. 1 und 3 schematische Blockschaltbilder von Beispielen von medizinischen Pumpen, die das Verständnis der Erfindung erleichtern,
- Fig. 2 schematisches Blockschaltbild einer erfindungsgemäßen Ausführungsform der Pumpe,
- Fig. 4 eine perspektivische Explosionsdarstellung einer Ausführungsform der Pumpe,
- Fig. 5 eine Seitenansicht der Pumpe nach Figur 4,
- Fig. 6 ein Schnitt entlang der Linie VI-VI aus Figur 5,
- Fig. 7 und 8 einen Teilschnitt durch die medizinische Pumpe im Bereich des Druckregelventils in verschiedenen Regelpositionen und
- Fig. 9 eine perspektivische Darstellung des Zylinderkopfes.

In der nachfolgenden Beschreibung werden für gleiche und gleich wirkende Teile dieselben Bezugsziffern verwendet.

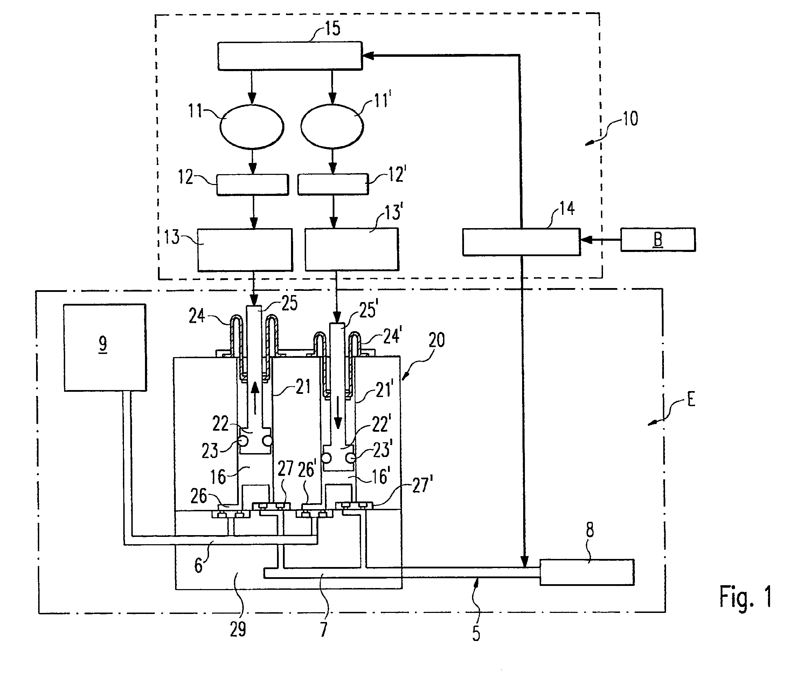

Bei dem in Figur 1 dargestellten Beispiel ist eine Pumpenbetätigungseinrichtung 10 vorgesehen, welche eine Motorsteuerung 15 zur Steuerung von zwei Motoren 11, 11' umfasst, die über Getriebe 12, 12' und Kupplungseinrichtungen 13, 13' mit Kolbenstangen 25, 25' verbunden sind. Eine Bedienungsperson B kann durch geeignete Schalteinrichtungen (Fußschalter, Fingerschalter) die Motorsteuerung 15 derart betätigen, dass die Motoren 11, 11' über die beschriebene Kette die Kolbenstangen 25, 25' und damit Kolben 22, 22' in Zylindern 21, 21' einer Pumpeneinheit 20 alternierend verschieben, sodass Druckräume 16, 16' der Pumpeneinheit 20 alternierend in ihrem Volumen vergrößert und verkleinert werden.

Zur Abdichtung der Druckräume 16, 16' bzw. der Kolben 22, 22' gegenüber den Zylindern 21, 21' sind an den Kolben 22, 22' Dichtungen 23, 23' vorgesehen. Darüber hinaus sind die Kolbenstangen 25, 25' über Rollmembranen 24, 24', welche einerseits mit den Zylindern 21, 21' und andererseits mit den Kolbenstangen 25, 25' fest verbunden sind, keimdicht abgedichtet. Auf diese Weise können Keime, die sich aus der Umgebungsluft ohne diese Rollmembranen 24, 24' an den Innenwänden der Zylinder 21, 21' absetzen und von den Dichtungen 23, 23' durchgelassen werden, nicht mit dem Arbeitsfluid vermischen bzw. in dieses gelangen.

Mit den Druckräumen 16, 16' verbunden sind Saugventile 26, 26' sowie Druckventile 27, 27'. Die Saugventile 26, 26' stehen über einen Fluideinlass 6 mit einem Vorratsbehälter 9 für das Arbeitsfluid in Verbindung. Die Druckventile 27, 27' stehen über einen Fluidauslass 7 mit einem Druckschlauch 5 in Verbindung, der zu einem Applikator 8 führt. Die Pumpeinheit 20 bildet zusammen mit dem Vorratsbehälter 9 samt seinem Inhalt, dem Druckschlauch 5 und dem Applikator 8 ein Einwegteil E, dass nach jeder Operation entsorgt wird, sodass die Gesamtanordnung den allerhöchsten Sterilitätsanforderungen genügt.

Zum Einstellen des Druckes ist bei dieser einfachen, hinsichtlich dieses Merkmals nicht erfindungsgemäßen Ausführungsform ein Klemmventil 14 vorgesehen, über welches (zusätzlich zur Motorsteuerung 15) eine Einstellung des Fluidflusses durch die Bedienungsperson B geschehen kann.

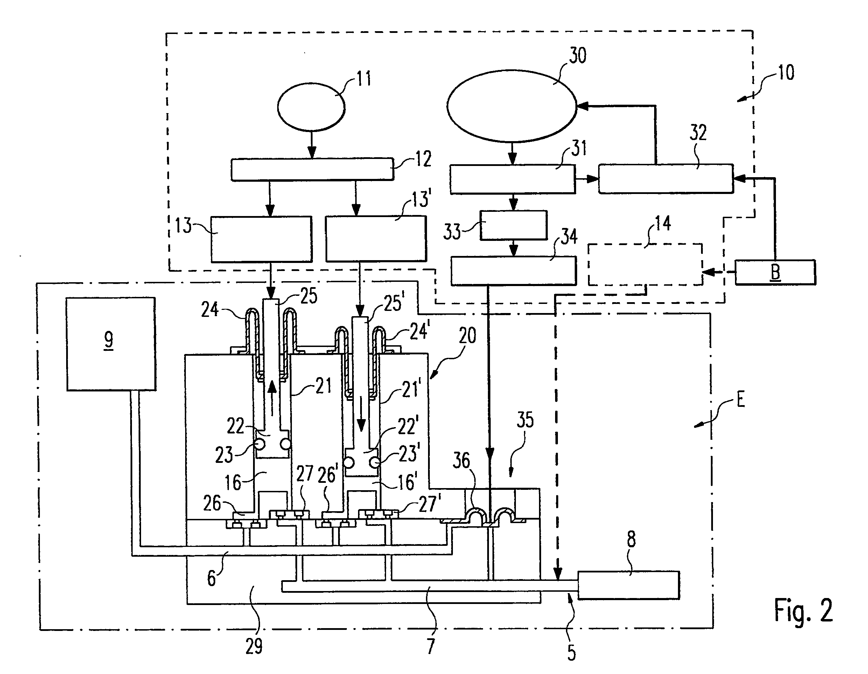

Die in Figur 2 gezeigte Ausführungsform der Erfindung unterscheidet sich von der nach Figur 1 dadurch, dass in erfindungsgemäßer Weise ein Druckregelventil 35 vorgesehen ist, das mittels einer Ventilmembran 36 einen Verbindungskanal zwischen dem Fluidauslass 7 und dem Fluideinlass 6 öffnen und schließen kann. Die Membran 36 wird über eine Stößelstange 34 und eine Feder 33 sowie einen Kraftmesser 31 von einem Stellmotor 30 betätigt. Der Kraftmesser 31 liefert ein kraftproportionales Ausgangssignal an einen Regler 32, über welchen eine Bedienungsperson B einen Maximaldruck vorgeben kann. Statt eines gesonderten Kraftmessers 31 kann auch ein Betätigungsstrom des Stellmotors 30 gemessen werden, der ebenfalls kraftproportional ist.

Durch diese Anordnung ist gewährleistet, dass der Fluiddruck am Applikator 8 exakt eingestellt werden kann. Darüber hinaus werden durch die Kolbenbetätigung auftretende

Druckschwankungen durch das Regelventil 35 ausgeglichen. Ein wesentlicher Punkt hierbei liegt darin, dass das Druckregelventil 35 aufgrund seiner Konstruktion mit einer vom Fluiddruck beaufschlagten Membran kraftgesteuert arbeitet und nicht weggesteuert. Dadurch kann bei einem Ankoppeln der Pumpeneinheit 20 an die Pumpenbetätigungseinrichtung 10 auch bei Maßtoleranzen kein Druckeinstellungsfehler auftreten, da es nicht auf die geometrischen Maße (also den Weg), sondern vielmehr auf die Kraft ankommt, mit welcher das Druckregelventil 35 betätigt wird.

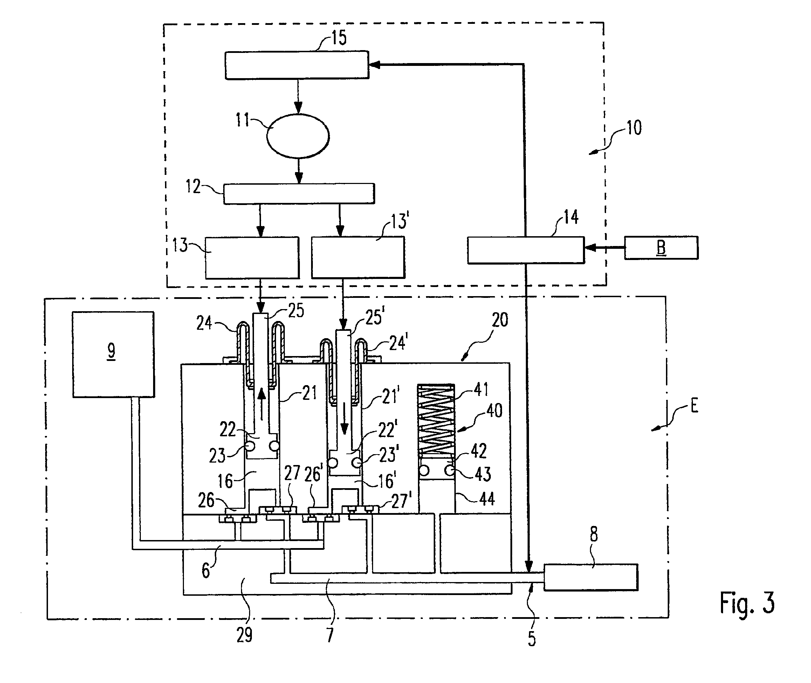

Das in Figur 3 gezeigte Beispiel unterscheidet sich von den zuvor gezeigten Pumpen dadurch, dass ein Druckspeicher 40 vorgesehen ist, der einen Zylinder 44 und in diesem einen über eine Dichtung 43 abgedichteten Kolben 42 umfasst, der von einer Feder 41 beaufschlagt wird. Der über den Kolben liegende Raum ist mit dem Fluidauslass verbunden, sodass bei steigendem Druck am Fluidauslass 7 die Feder 41 zusammengedrückt wird, während bei sinkendem Druck die Feder 41 den Kolben 42 antreibt. Auf diese Weise wird eine Vergleichmäßigung des dem Applikator 8 zugeführten Druckes nach Art einer Tiefpassfunktion erzielt. Dieser Druckspeicher 40 ist in einem Zylinderkopf 29 angeordnet, der die Zylinder 21, 21' abschließt.
Selbstverständlich ist es möglich, die hier gezeigten Varianten miteinander zu kombinieren. Insbesondere kann das Druckregelventil 35 mit dem Druckspeicher 40 kombiniert werden.

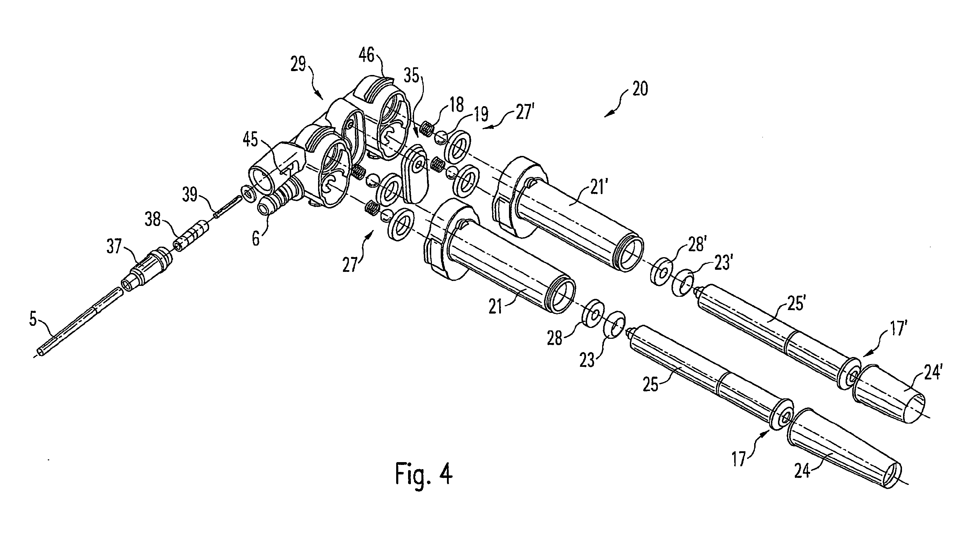

In Figur 4 ist eine konstruktive Ausführungsform der Pumpeinrichtung 20 in einer perspektivischen Explosionsdarstellung gezeigt. Bei dieser Ausführungsform umfassen die Druck- und Saugventile 26/27 Kugeln 19, die über Federn 18 auf Ventilsitze (in der Abbildung nicht sichtbar) gedrückt werden, wie dies im Prinzip bekannt ist.

Der Zylinderkopf 29 weist zwei Abschnitte zum Ankoppeln der Zylinder 21, 21' auf, wobei die Ventile zwischen den Zylindern 21, 21' und dem Zylinderkopf 29 sitzen.

Weiterhin ist aus der Darstellung nach Figur 4 ersichtlich, dass die Kolbenstangen 25, 25' an ihren distalen Enden Koppelvorsprünge 17, 17' aufweisen, über welche eine mechanische Verbindung mit den Kupplungssystemen 13, 13' bewerkstelligt wird.

Die Kolben werden bei dieser Ausführungsform der Erfindung durch proximale Enden der Kolbenstangen 25, 25' mit aufgesetzten Kappen 28 gebildet, welche gleichzeitig die Dichtungen 23, 23' fest auf den Kolbenstangen 25, 25' halten.

Der Druckschlauch 5 wird am Zylinderkopf 29 über einen Überwurfstutzen 37, ein Crimprohr 38 und ein in den Druckschlauch 5 einzusetzendes Innenrohr irreversibel befestigt, wobei nach einem Zusammenstecken (in an sich bekannter Weise) der Überwurfstutzen 37 im Zylinderkopf 29 mittels einer Schnappzunge 45 gehalten wird, die den Überwurfstutzen 37 im Zylinderkopf 29 irreversibel hält.

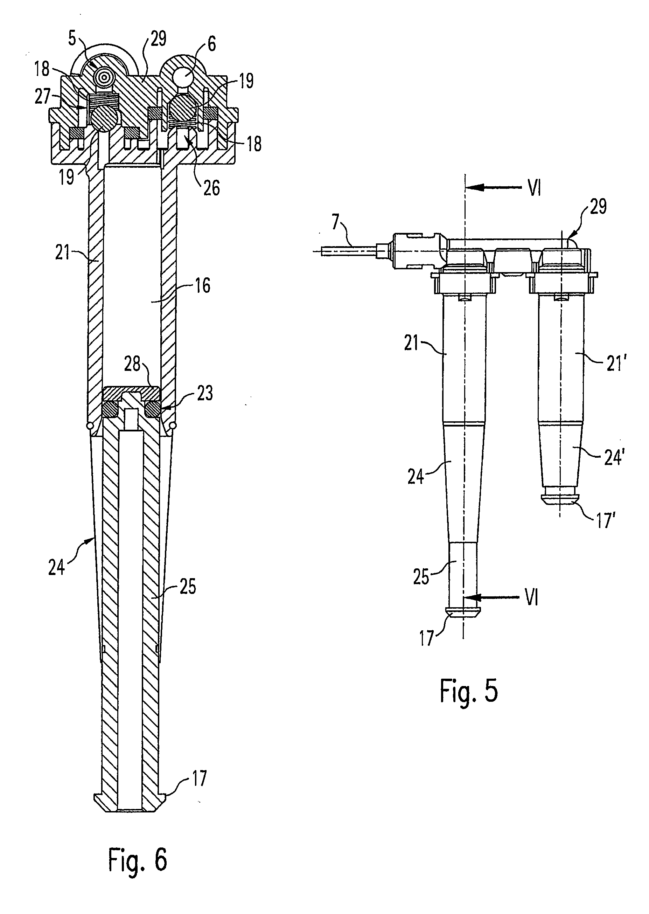

Aus den Figuren 5 und 6 sind Details der Anordnung insbesondere in Bezug auf die Konstruktion des Saugventil 26, 26' bzw. des Druckventils 27, 27' ersichtlich, insbesondere die Anordnung der Ventilsitze einerseits im Zylinderkopf 29 und andererseits in den jeweiligen zugeordneten Zylindern 21, 21'.

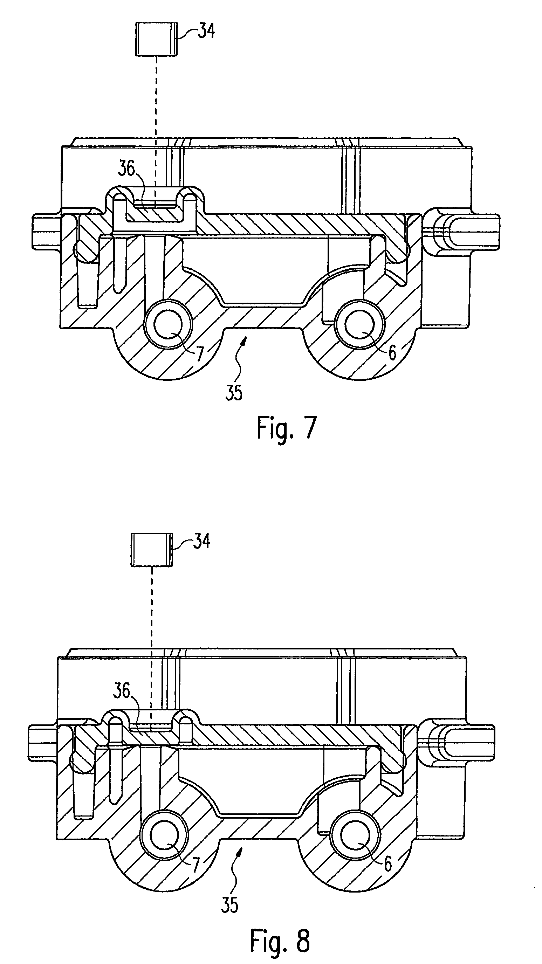

In den Figuren 7 und 8 ist ein Schnitt durch das Druckregelventil 35 gezeigt, aus dem hervorgeht, dass die Membran 36 durch die Stößelstange 34 auf einen Ventilsitz gepresst werden kann (Figur 7 zeigt den geöffneten, Figur 8 den geschlossenen Zustand), sodass zwischen dem Fluidauslass 7 und dem Fluideinlass 6 je nach Position der Membran 36 ein mehr oder minder großer "Kurzschluss" der Pumpeinheit 20 erzeugt wird. Da die Membran 36 mit dem Druck im Fluidauslass 7 beaufschlagt wird, liegt hier ein kraftgesteuertes Ventil vor.

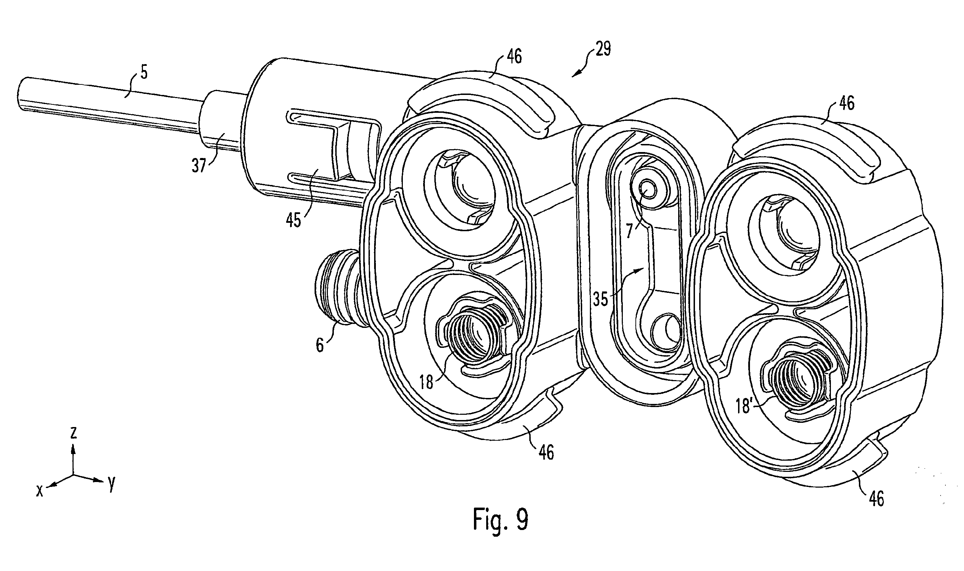

Aus Figur 4 gehen weitere konstruktive Details des Zylinderkopfes 29 und der darin enthaltenden Ventileinrichtungen (Saugventil, Druckventil und Druckregelventil) hervor. Darüber hinaus sind in Figur 9 auch Haltevorsprünge 46 gezeigt, über welche die Pumpeinheit 20 an die Pumpenbetätigungseinrichtung 10 angekoppelt bzw. fest auf dieser gehalten werden können.

Bei einer hier nicht gezeigten Ausführungsform der Erfindung ist nicht nur das Druckregelventil 35 als Membranventil ausgebildet, vielmehr sind auch die beiden Druckventile 27, 27' bzw. Saugventile 26, 26' als Membranventile anstelle der hier gezeigten Kugelventile ausgebildet. Dadurch wird die Anordnung noch kostengünstiger. Es ist schließlich auch möglich, die Anordnung derart zu treffen, dass nicht nur sämtliche Ventile als Membranventile ausgebildet sind, vielmehr können alle Membranen miteinander einstückig verbunden sein, sodass die Anzahl der Teile weiter sinkt.

### Bezugszeichenliste

- E: Einwegteil
- B: Bedienungsperson
- 5: Druckschlauch
- 6: Fluideinlass
- 7: Fluidauslass
- 8: Applikator
- 9: Vorratsbehälter
- 10: Pumpenbetätigungseinrichtung
- 11, 11': Motor
- 12, 12': Getriebe
- 13, 13': Kupplungssystem
- 14: Klemmventil
- 15: Motorsteuerung
- 16, 16': Druckraum
- 17, 17': Koppelvorsprung
- 18: Feder
- 19: Kugel
- 20: Pumpeinheit
- 21, 21': Zylinder
- 22, 22': Kolben
- 23, 23': Dichtung
- 24, 24': Rollmembran
- 25, 25': Kolbenstange
- 26, 26': Saugventil
- 27, 27': Druckventil
- 28: Kappe
- 29: Zylinderkopf
- 30: Stellmotor
- 31: Kraftmesser
- 32: Regler
- 33: Feder
- 34: Stößelstange
- 35: Druckregelventil
- 36: Ventil-Membran
- 37: Überwurfstutzen
- 38: Crimprohr
- 39: Innenrohr
- 40: Druckspeicher
- 41: Feder
- 42: Kolben
- 43: Dichtung
- 44: Zylinder
- 45: Schnappzunge
- 46: Haltevorsprünge

## Patentansprüche

1. Wasserstrahlchirurgiepumpe,
mit einem einstellbaren Druckregelventil (35), um den Druck an einem Fluidauslass (7) auf einen voreinstellbaren Maximalwert zu begrenzen, **gekennzeichnet durch**
mindestens zwei Kolben (22, 22') mit Kolbenstangen (25, 25') zum Verschieben der Kolben (22, 22') in Zylindern (21, 21') und zum Ankoppeln an eine Pumpenbetätigungseinrichtung (10);
einen Zylinderkopf (29) zum Abschließen der Zylinder (21, 21') und
Ventileinrichtungen (26, 26'; 27, 27') zum Verbinden jeweils eines Druckraumes (16, 16') in den Zylindern (21, 21') mit mindestens einem Fluideinlass (6) und mindestens einem Fluidauslass (7),
wobei das Druckregelventil (35) den Fluideinlass (6) mit dem Fluidauslass (7) verbindend angeordnet ist.

2. Wasserstrahlchirurgiepumpe nach Anspruch 1,
**dadurch gekennzeichnet, dass**
die Ventileinrichtungen (26, 27) und/oder das Druckregelventil (35) eine elastische oder elastisch beaufschlagte Ventil-Membran (36) umfassen.

3. Wasserstrahlchirurgiepumpe nach einem der vorhergehenden Ansprüche,
**dadurch gekennzeichnet, dass**
die Ventileinrichtungen (26, 27) federbelastete Kugel-Rückschlagventile umfassen.

4. Wasserstrahlchirurgiepumpe nach einem der vorhergehenden Ansprüche,
**dadurch gekennzeichnet, dass**
das Druckregelventil (35) derart als kraftgesteuertes Ventil ausgebildet ist, dass der Maximalwert durch eine auf ein Stellglied (36) des Druckregelventils (35) wirkende Stellkraft einstellbar ist.

5. Wasserstrahlchirurgiepumpe nach einem der vorhergehenden Ansprüche,
**dadurch gekennzeichnet, dass**
die Kolben (22, 22') oder die Kolbenstangen (25, 25') über einen Balg, eine Rollmembran oder dergleichen gleitfreie Dichtung (24, 24') mit den Zylindern (21, 21') dicht und keimundurchlässig verbunden sind.

6. Wasserstrahlchirurgiepumpe nach einem der vorhergehenden Ansprüche,
**dadurch gekennzeichnet, dass**
die Ventileinrichtungen (26, 27) und/oder das Druckregelventil (35) mindestens teilweise im Zylinderkopf (29) angebracht sind.

7. Wasserstrahlchirurgiepumpe nach einem der vorhergehenden Ansprüche,
**dadurch gekennzeichnet, dass**
die Zylinder (21, 21') einzeln mit dem Zylinderkopf (29) verbindbar sind.

8. Wasserstrahlchirurgiepumpe nach einem der vorgehenden Ansprüche,
**dadurch gekennzeichnet, dass**
der Fluidauslass (7) Verbindungseinrichtungen (37-39, 45) zum irreversiblen Verbinden mit einem Druckschlauch (5) aufweist.

9. Wasserstrahlchirurgiepumpe nach einem der vorhergehenden Ansprüche,
**dadurch gekennzeichnet, dass**
der Zylinderkopf (29) Halteeinrichtungen, insbesondere Haltevorsprünge (46) zum Einrasten von Halteklinken aufweist, die an der Pumpenbetätigungseinrichtung (10) angebracht sind.

10. Wasserstrahlchirurgiepumpe nach einem der vorhergehenden Ansprüche,
**gekennzeichnet durch**
eine Druckspeichereinrichtung (40), die derart ausgebildet und mit dem Fluidauslass (7) verbunden ist, dass Druckschwankungen des Fluids am Fluidauslass (7) nach Art einer Tiefpassfunktion ausgleichbar sind.

11. Wasserstrahlchirurgiepumpe nach Anspruch 10,
**dadurch gekennzeichnet, dass**
die Druckspeichereinrichtung (40) im Zylinderkopf (29) angeordnet oder mit diesem verbunden ist.

## Claims

1. Waterjet surgery pump,
with an adjustable pressure control valve (35) for limiting the pressure at a fluid outlet (7) to a predefinable maximum value,
**characterized by**
at least two pistons (22, 22') with piston rods (25, 25') for moving the pistons (22, 22') in cylinders (21, 21') and for coupling to a pump-actuating device (10);
a cylinder head (29) for sealing off the cylinders (21, 21'), and
valve devices (26, 26'; 27, 27') for respectively connecting a pressure chamber (16, 16') in the cylinders (21, 21') to at least one fluid inlet (6) and at least one fluid outlet (7),
wherein the pressure control valve (35) is arranged connecting the fluid inlet (6) to the fluid outlet (7).

2. Waterjet surgery pump according to Claim 1, **characterized in that** the valve devices (26, 27) and/or the pressure control valve (35) comprise an elastic or elastically actuated valve membrane (36).

3. Waterjet surgery pump according to one of the preceding claims, **characterized in that** the valve devices (26, 27) comprise spring-loaded non-return ball valves.

4. Waterjet surgery pump according to one of the preceding claims, **characterized in that** the pressure control valve (35) is designed as a force-controlled valve, in such a way that the maximum value can be set by an adjusting force acting on an adjusting member (36) of the pressure control valve (35).

5. Waterjet surgery pump according to one of the preceding claims, **characterized in that** the pistons (22, 22') or the piston rods (25, 25') are connected to the cylinders (21, 21'), in a leaktight and germ-proof manner, by a bellows, a rolling diaphragm or similar non-slide seal (24, 24').

6. Waterjet surgery pump according to one of the preceding claims, **characterized in that** the valve devices (26, 27) and/or the pressure control valve (35) are mounted at least partially in the cylinder head (29).

7. Waterjet surgery pump according to one of the preceding claims, **characterized in that** the cylinders (21, 21') can be connected individually to the cylinder head (29).

8. Waterjet surgery pump according to one of the preceding claims, **characterized in that** the fluid outlet (7) has connecting devices (37-39, 45) for irreversible connection to a pressure hose (5).

9. Waterjet surgery pump according to one of the preceding claims, **characterized in that** the cylinder head (29) has holding devices, in particular holding projections (46) for the engagement of detent latches, which are mounted on the pump-actuating device (10).

10. Waterjet surgery pump according to one of the preceding claims, **characterized by** a pressure accumulator (40), which is designed in such a way and connected to the fluid outlet (7) in such a way that fluctuations in the pressure of the fluid at the fluid outlet (7) can be levelled out in the manner of a low-pass function.

11. Waterjet surgery pump according to Claim 10, **characterized in that** the pressure accumulator (40) is arranged in the cylinder head (29) or is connected thereto.

## Revendications

1. Pompe pour la chirurgie à jet d'eau,
comprenant une soupape de régulation de pression réglable (35) afin de limiter la pression au niveau d'une sortie de fluide (7) à une valeur maximale préréglable,
**caractérisée par**
au moins deux pistons (22, 22') pourvus de tiges de piston (25, 25') en vue du déplacement des pistons (22, 22') dans des cylindres (21, 21') et en vue de l'accouplement à un dispositif d'actionnement de pompe (10) ;
une tête de cylindres (29) pour fermer les cylindres (21, 21') et
des dispositifs de soupape (26, 26' ; 27, 27') pour relier respectivement un espace de pression (16, 16') dans les cylindres (21, 21') à au moins une entrée de fluide (6) et à au moins une sortie de fluide (7),
la soupape de régulation de pression (35) étant disposée de manière à relier l'entrée de fluide (6) à la sortie de fluide (7).

2. Pompe pour la chirurgie à jet d'eau selon la revendication 1,
**caractérisée en ce que**
les dispositifs de soupape (26, 27) et/ou la soupape de régulation de pression (35) comportent une membrane de soupape (36) élastique ou sollicitée élastiquement.

3. Pompe pour la chirurgie à jet d'eau selon l'une quelconque des revendications précédentes,
**caractérisée en ce que**
les dispositifs de soupape (26, 27) comportent des soupapes anti-retour à bille sollicitées par ressort.

4. Pompe pour la chirurgie à jet d'eau selon l'une quelconque des revendications précédentes,
**caractérisée en ce que**
la soupape de régulation de pression (35) est réalisée sous forme de soupape commandée par force, de telle sorte que la valeur maximale puisse être réglée au moyen d'une force de réglage agissant sur un organe de réglage (36) de la soupape de régulation de pression (35).

5. Pompe pour la chirurgie à jet d'eau selon l'une quelconque des revendications précédentes,
**caractérisée en ce que**
les pistons (22, 22') ou les tiges de piston (25, 25') sont relié(e)s aux cylindres (21, 21') de manière étanche et imperméable aux germes par l'intermédiaire d'un soufflet, d'une membrane déroulante ou d'un joint d'étanchéité (24, 24') sans glissement similaire.

6. Pompe pour la chirurgie à jet d'eau selon l'une quelconque des revendications précédentes,
**caractérisée en ce que**
les dispositifs de soupape (26, 27) et/ou la soupape de régulation de pression (35) sont montés au moins partiellement dans la tête de cylindres (29).

7. Pompe pour la chirurgie à jet d'eau selon l'une quelconque des revendications précédentes,
**caractérisée en ce que**
les cylindres (21, 21') peuvent être reliés individuellement à la tête de cylindres (29).

8. Pompe pour la chirurgie à jet d'eau selon l'une quelconque des revendications précédentes,
**caractérisée en ce que**
la sortie de fluide (7) comprend des dispositifs de liaison (37-39, 45) en vue d'une liaison irréversible à un tuyau sous pression (5).

9. Pompe pour la chirurgie à jet d'eau selon l'une quelconque des revendications précédentes,
**caractérisée en ce que**
la tête de cylindres (29) comprend des dispositifs de retenue, en particulier des saillies de retenue (46) pour l'encliquetage de cliquets de retenue qui sont montés sur le dispositif d'actionnement de pompe (10).

10. Pompe pour la chirurgie à jet d'eau selon l'une quelconque des revendications précédentes,
**caractérisée par**
un dispositif de réservoir sous pression (40) qui est relié à la sortie de fluide (7) et réalisé de telle sorte que les fluctuations de la pression du fluide au niveau de la sortie de fluide (7) puissent être compensées à la manière d'une fonction passe-bas.

11. Pompe pour la chirurgie à jet d'eau selon la revendication 10,
**caractérisée en ce que**
le dispositif de réservoir sous pression (40) est disposé dans la tête de cylindres (29) ou est relié à celle-ci.
